# EUROPÄISCHE PATENTSCHRIFT

(11) **EP 2 807 134 B1**
(45) Veröffentlichungstag und Bekanntmachung des Hinweises auf die Patenterteilung: **29.08.2018**
(21) Anmeldenummer: 13701618.4
(22) Anmeldetag: 24.01.2013
(51) Int. Cl.: C07C 1/24, C07C 11/04

(54) **VERFAHREN ZUR HERSTELLUNG VON ETHYLEN UND ANDEREN OLEFINEN AUS WÄSSRIGEN LÖSUNGEN DER KORRESPONDIERENDEN ALKOHOLE**
PROCESS FOR PREPARING ETHYLENE AND OTHER OLEFINS FROM AQUEOUS SOLUTIONS OF THE CORRESPONDING ALCOHOLS
PROCÉDÉ DE PRODUCTION D'ÉTHYLÈNE ET D'AUTRES OLÉFINES À PARTIR DE SOLUTIONS AQUEUSES DES ALCOOLS CORRESPONDANTS

(30) Priorität: 24.01.2012 DE 102012200996
(43) Veröffentlichungstag der Anmeldung: 03.12.2014
(73) Patentinhaber: Fraunhofer-ges. zur Förderung der Angewandten Forschung E.V., 80686 München (DE)
(72) Erfinder: UNKELBACH, Gerd, 04179 Leipzig (DE); SCHWEPPE, Rainer, 76228 Karlsruhe (DE); HIRTH, Thomas, 77815 Bühl (DE)
(74) Vertreter: Strehl Schübel-Hopf & Partner
(86) Internationale Anmeldenummer: PCT/EP2013/051367
(87) Internationale Veröffentlichungsnummer: WO 2013/110723

(56) Entgegenhaltungen:
- WO-A1-2009/022990
- WO-A1-2009/098269
- GB-A- 576 480
- NOURI S. AND AMRAN T.: "Supercritical water ethanol reforming for hydrogen production: (effect of different support on nickel based catalyst in supercritical water conditions)", ASIAN JOURNAL OF CHEMISTRY, Bd. 22, 2010, Seiten 569-581, XP002693755,
- MASARU WATANABE ET AL: "Conversions of some small organic compounds with metal oxides in supercritical water at 673 KPresented at The First International Conference on Green & Sustainable Chemistry, Tokyo, Japan, March 13?15, 2003.", GREEN CHEMISTRY, Bd. 5, Nr. 5, 1. Januar 2003 (2003-01-01), Seite 539, XP55056339, ISSN: 1463-9262, DOI: 10.1039/b304686a in der Anmeldung erwähnt
- RAMAYYA S ET AL: "Acid-catalysed dehydration of alcohols in supercritical water", FUEL, IPC SCIENCE AND TECHNOLOGY PRESS, GUILDFORD, GB, Bd. 66, Nr. 10, 1. Oktober 1987 (1987-10-01), Seiten 1364-1371, XP025417215, ISSN: 0016-2361, DOI: 10.1016/0016-2361(87)90183-9 [gefunden am 1987-10-01]

## Beschreibung

Die Erfindung betrifft ein Verfahren zum Herstellen von Ethylen und ggf. anderen Olefinen aus wässrigen Lösungen der korrespondierenden Alkohole in komprimierter Phase unter Verwendung eines heterogenen Katalysators. Das Verfahren eignet sich insbesondere für kontinuierlich betriebene Reaktorsysteme.

Vor 1945 basierte der Großteil der Ethylenproduktion auf der Dehydratisierung von Ethanol in der Gasphase an heterogenen Katalysatoren. Die Reaktion wurde in Wirbelbettraktoren bei 350-400°C durchgeführt, die Kontaktzeiten zwischen Katalysator und Edukt lagen bei 1-10 Sekunden. Als Katalysatoren wurden "saure" Feststoffe, z.B. Silika-Aluminiumoxid-Katalysatoren, oder verschiedenste Zeolithe verwendet. Die Ausbeuten lagen bei 99% (US 134,926), die Selektivität bei 98%. Später wurde das Steamcracking von relativ kostengünstigen Kohlenwasserstoffen das Verfahren der Wahl.

Aus dem Jahre 1944 stammt ein Vorschlag zur katalytischen Dehydratisierung von Ethanol unter Temperaturen von oberhalb 250°C und beliebigen, hohen Drücken (GB 587,378). Als mögliche Katalysatoren werden Phosphorsäure, konzentrierte Schwefelsäure, γ-Aluminiumoxid und Natriumcarbonat genannt; das einzige Ausführungsbeispiel zeigt die Umsetzung von Alkohol in Gegenwart von 5% Phosphorsäure im Batch-Verfahren. Nach Abtrennen des bei der Reaktion entstehenden Wassers sei das gebildete Ethylen ausreichend rein für eine Reihe von - nicht genannten - Zwecken.

Heute erlangt die Dehydratisierung von Ethanol neues Interesse, weil Ethanol in großen Mengen durch die Fermentierung von Biomasse anfällt. Die Gasphasendehydratisierung kommt hierfür jedoch kaum in Frage, weil dafür reines Ethanol benötigt wird, weshalb das Wasser durch Rektifikation aus der Fermenterlösung entfernt und anschließend aus dem Azeotrop entfernt werden muss. Der Energie- und Kostenaufwand für ein solches Verfahren ist derart hoch, dass es in den meisten Industrienationen nicht als realistische Alternative in Betracht gezogen wird.

In WO 2007/003899 A1, WO 2007/003901 und WO 2007/003910 A1 wird ein Verfahren zum Herstellen von Olefinen, darunter vorwiegend Ethylen beschrieben, das die Anwesenheit von bis zu 50 Gew.-% Wasser erlaubt. Bei höheren Wassergehalten, z.B. im Falle von nicht aufbereitetem Bioalkohol, sollte eine Destillation vorgeschaltet werden, um den Wassergehalt zu senken. Bei erhöhtem Druck und erhöhter Temperatur, die nicht näher charakterisiert sind, soll das Ausgangsmaterial umgesetzt werden; es entstehen Alkene und Ether, die anschließend getrennt werden. Die Konkurrenz der zugrundeliegenden Reaktionen wird diskutiert; ein zu geringer Anteil an Ethylen könne durch eine Kombination von Reaktion und Destillation ausgeglichen werden. Als Katalysatoren werden gleichermaßen heterogene und homogene Katalysatoren vorgeschlagen, darunter Zeolithe, sulfonierte Träger, Schwefel- und Phosphorsäure. Heteropolysäuren werden als bevorzugt genannt. Im einzigen Beispiel wird eine 80%ige wässrige Ethanollösung ohne Katalysator einer reaktiven Destillation unterworfen. In WO 2011/115656 wird festgestellt, dass bei der Ethanol-Dehydratisierung der Wechsel des verwendeten Katalysators von reinem Aluminiumoxid zu Aluminiumoxid im Gemisch mit Yttriumoxid die Entstehung von oxygenierten Produkten senkt und dass unter diesen Produkten vorwiegend das leichter abtrennbare CO₂ entsteht, während die Bildung von Acetaldehyd unter der Nachweisgrenze liegt.
Es existiert eine Reihe von Untersuchungen der Umsetzung von Propanol in überkritischem Wasser. M. Watanabe et al. stellen in Green Chemistry, 2003(5), 539-544 Ergebnisse vor, die sie bei Verwendung von Katalysatoren erhielten. Demnach fördert Schwefelsäure die Bildung von Propylen aus 2-Propanol, während in Gegenwart von NaOH die Bildung von Aceton bevorzugt ist und gar kein Propylen entsteht. Mit verschiedenen Metalloxiden als Katalysatoren entstand immer Propylen, weshalb die Autoren in überkritischem Wasser saure Zentren an diesen Materialien vermuten. Die Ausbeute war jedoch von Oxid zu Oxid sehr unterschiedlich, mit ZrO₂ und TiO₂ entstand daneben auch Aceton (die Messungen erfolgten meist nach 15 min Reaktionsdauer). Die gefundene Spezifität war für Schwefelsäure und die meisten der eingesetzten Oxide vergleichbar und lag bei etwa 70 mol-%. V.l. Anikeev et al. untersuchten das kinetische und thermodynamische Verhalten von 2-Propanol (The Journal of Supercritical Fluids 32, 123-135 (2004)), aber nur in Hinblick auf den Einfluss der Dichte des Reaktionsmediums.
Während sich in überkritischem Wasser Propanol relativ problemlos in Propen überführen lässt, gelingt die Analogreaktion mit Ethanol nicht ohne weiteres. Der Übersichtsbeitrag von G. Herbert Vogel in Chemie Ingenieur Technik 2011, 83, S. 1-9 bezeichnet diesen Alkohol als "härteste Nuss". Dabei liegt das Problem teils in den geringen Reaktionsumsätzen, teils in der geringen Spezifität. Die Umsetzung von Ethanol in überkritischem Wasser wird in Asian Journal of Chemistry, 2010(22), 569-581 beschrieben. Xiadong Xu et al. untersuchten die Dehydratisierung von Ethanol bei verschiedenen Konzentrationen des Alkohols in Wasser, verschiedenen Schwefelsäurekonzentrationen und verschiedenen Verweilzeiten (The Journal of Supercritial Fluids, 1990(3), 228-232; Ind. Eng. Chem. Res. 30(7), 1991, 1478-1485). Die Ergebnisse zeigen, dass in Gegenwart von Schwefelsäure die Dehydratisierung zu Ethanol zwar eine hohe Spezifität besitzen solle; die Ausbeuten seien jedoch unbefriedigend. Zu ähnlichen Ergebnissen kommen S. Ramayya et al. in FUEL 66 (1987), 1364-1371; bei den von dieser Gruppe vorgestellten Ergebnissen ist zu beachten, dass die Kohlenstoffbilanz im Experiment nur zu 88% geschlossen werden konnte, obwohl keine Nebenprodukte detektiert wurden. Bei einer Steigerung der Ethanolkonzentration im Zustrom zum Reaktor steigt demnach die Ethylenausbeute nur langsam, während die Diethyletherbildung sehr stark zunimmt. Deshalb wird in der Literatur zur Verbesserung der Bilanz eine Rückführung des entstehenden Ethers bei höheren Ethanolkonzentrationen in den Reaktor vorgeschlagen (Halvorsen et al. in Process Optimization for the Supercritical Dehydration of Ethanol to Ethylene in AIChENational Meeting, Chicago, 1990).

Schwefelsäure wie auch Aluminiumsulfat als Katalysator ist in vielen Fällen korrosiv; die angegriffenen Behälterwände können dann ihrerseits katalytische Wirksamkeit entfalten, wie G. Aras et al. in einem Abschlussbericht für die Max-Buchner-Forschungsstiftung im Januar 2011 mit dem Titel "Reaktionstechnische Untersuchungen zur Ethylenherstellung aus wässrigen Ethanollösungen (Fermenterausträgen) - Eine nachhaltige Alternative zum Steam-Cracker" (Technische Universität Darmstadt) zeigen konnten. Die von dieser Gruppe ermittelten Umsätze mit verschiedenen anderen homogenen Katalysatoren lagen nie über 10%; insbesondere erwies sich Zinksulfat, das die Dehydratisierung von Propanol zu Propen sehr wirksam katalysiert, als völlig ungeeignet. Das bestätige die Vermutung, dass Ethanol aufgrund des weniger stabilisierten Carbokations am schwierigsten zu dehydratisieren ist. Eine Erhöhung der Temperaturen oder Verweildauern über die untersuchten hinaus sei nicht angezeigt, weil dann die Vergasungsreaktion zu Synthesegas zu dominieren beginne.

Zusammenfassend lässt der zur Dehydratisierung von Alkoholen in überkritischem Wasser existierende Stand der Technik den Schluss zu, dass (a) flüssige Säuren als Katalysatoren wegen konkurrierender Reaktion an der Behälterwand nur schwer zu kontrollieren sind und (b) die Dehydratisierung von Ethanol mit derjenigen höherer Alkohole nicht verglichen werden kann, da die Ausbeuten und insbesondere Spezifitäten aufgrund der unterschiedlichen elektronischen Situation in den Übergangszuständen bei höheren Säuren wesentlich besser sind. Die rein spekulativen Behauptungen der älteren Druckschrift GB 587 378 und die widersprüchlichen Ergebnisse jüngerer Untersuchungen lassen den Fachmann im Übrigen ohne klares Bild.

Der Erfindung liegt die Aufgabe zugrunde, ein kontinuierlich durchführbares Verfahren aufzufinden, mit dem sich aus alkoholisch-wässrigen Lösungen auch geringer Konzentration und insbesondere solchen, die Ethanol enthalten, durch Dehydratisierung kosteneffizient und unaufwändig das oder die entsprechende(n) Alken(e) gewinnen lassen, wobei das Verfahren insbesondere eine hohe Spezifität der Dehydratisierung im Verhältnis zu Konkurrenzreaktionen ermöglichen soll. Auch wird das Entstehen von ausschließlich umweltfreundlich entsorgbaren Rückständen angestrebt.

Zur Lösung der Aufgabe schlagen die Erfinder ein Verfahren unter Reaktionsbedingungen oberhalb des kritischen Punkt der Reaktionsmischung vor, und zwar unter Verwendung eines Festbettkatalysators mit spezifischen Eigenschaften. Die Wahl des Festbettkatalysators ermöglicht die unaufwändige Durchführung des Verfahrens unabhängig von der Art des Alkohols - und damit auch die Nutzung des Verfahrens für die Dehydratisierung von Ethanol. Der Festbettkatalysator, der in einem geeigneten Reaktor angeordnet ist, kann mit großen Mengen an Ausgangsmaterial durchströmt werden. Als Ausgangsmaterial dient eine wasserhaltige bzw. wässrige alkoholische Lösung, z.B. aus Biomasse, die insbesondere fermentiert sein kann. Da ausschließlich ein fester Katalysator verwendet wird und deshalb keine flüssige oder gelöste Säure in die umzusetzende bzw. umgesetzte Lösung gelangt, sind Trennschritte oder andere Aufreinigungen des Produktstroms nicht vonnöten und der Rückstand besitzt einen pH-Wert, der ohne weitere Maßnahmen eine umweltfreundliche Entsorgung ermöglicht.
Die Erfinder haben völlig überraschend festgestellt, dass die für das Verfahren vorgesehenen Festbettkatalysatoren bei der Wahl geeigneter, relativ kurzer Verweildauern sehr hohe Umsätze fast ohne Nebenreaktionen ermöglichen. Im überkritischen Bereich lassen sich Umsätze von fast 95% mit einer fast vollständigen Spezifität erzielen. Der Grund könnte darin gesehen werden, dass aufgrund der geringen Viskosität des Reaktionsgemischs bei gleichzeitig herrschender enorm hoher Dichte im überkritischen Zustand sehr hohe Diffusionsraten erzielt werden können. Dies ermöglicht eine hohe Raum-/Zeit-Ausbeute, was zu einer höheren Produktivität führt (bzw. die Verkleinerung des Reaktorvolumens ermöglicht). Auch besitzt Wasser unter überkritischen Bedingungen gute Lösungseigenschaften für unpolare Substanzen wie Olefine, da die relative Dielektrizitätskonstante stark abnimmt. Daher nehmen die Erfinder an, ohne darauf beschränkt werden zu wollen, dass sich Edukte, Zwischenstufen der Umsetzung und die resultierenden Olefine immer in einer homogenen, komprimierten Phase befinden und keine Diffusionshemmung eintritt. Nur dieser Zustand erlaubt den Einsatz stark wässriger Edukte, wie von der Erfindung angestrebt.
Als Katalysatoren für dieses Verfahren eignen sich Metalloxide, die sich wie Bronstedt-Säuren verhalten, d.h. Protonen-Donatoren sind, und darunter insbesondere solche, deren Oberfläche mit Säuregruppen modifiziert ist. Geeignet sind weiterhin unlösliche Metall- oder Halbmetallphosphate und Materialien mit einer hohen spezifischen Oberfläche, die mit anorganischen Säuremolekülen belegt sind, sofern diese Materialien unter den während der Umsetzung herrschenden Umgebungsbedingungen nicht angegriffen werden. Diese kennt der Fachmann; geeignet sind beispielsweise modifizierter Bims und modifizierte Aktivkohle, jedoch keine Zeolith-Materialien.

Als Metalloxide eignen sich Metalloxide, die die Eigenschaften einer Brønsted-Säure haben und wobei die Metalloxide Oxide von Kationen der 3. Hauptgruppe sowie der 4. bis 6. Nebengruppe der Periodensystems sind. Als Säuregruppen eignen sich insbesondere Sulfat- oder Phosphatgruppen. Geeignet sind beispielsweise Oxide des Aluminiums, Zirkoniums oder Titans, deren Oberfläche mit Säuregruppen belegt ist, oder poröse Materialien wie Bimsstein oder Kohlen, insbesondere Aktivkohle, deren Oberfläche mit Säuregruppen belegt ist. Die Belegung erfolgt bei diesen Materialien wiederum besonders bevorzugt mit Sulfat- oder Phosphatgruppen.

Es sei darauf hingewiesen, dass der Ausdruck "Metalloxide" sowohl unmodifizierte als auch (z.B. mit Säuregruppen) modifizierte Materialien umfassen soll.
Das Verfahren eignet sich insbesondere für die Umsetzung und damit Verwertung von Ethanol, ist aber nicht hierauf beschränkt, denn es eignet sich natürlich auch für die Umsetzung von höheren Alkoholen wie Propanol, oder Butanol. Häufig werden Ausgangsmaterialien eingesetzt, die Alkoholgemische enthalten. Diese können beispielsweise einen gewissen Anteil (z.B. mindestens 10 Gew.-% vorzugsweise mindestens 20 Gew.-%, stärker bevorzugt mindestens 50 Gew.-%) an Ethanol, bezogen auf das Gewicht der Alkohole, aufweisen; sie können aber auch ethanolfrei sein.

Die Umsetzung erfolgt bei mindestens 300°C, vorzugsweise mindestens bei 350°C, insbesondere im Falle der Umsetzung von Ethanol. Ebenfalls bevorzugt ist es, die Obergrenze von 600°C nicht zu überschreiten. Der Mindestdruck sollte ca. 220 bar (2,2 x 10⁷ Pa) betragen. Druck und Temperatur sollten so gewählt werden, dass die alkoholische Ausgangslösung ihren überkritischen Zustand erreicht und das Gemisch diesen Zustand auch im Zuge der Veränderung der Relation von Wasser zu Alkohol durch die Umsetzung von Alkohol zu Olefin beibehält. Günstig sind - in Abhängigkeit von der Alkohol-Konzentration - Temperaturen von 350°C oder darüber, vorzugsweise von 400°C oder darüber, jeweils bei Drücken von 250 bar oder darüber.
Die Ausgangskonzentration des Alkohols in der wässrigen Lösung kann in einem weiten Bereich variieren, der insbesondere 5 bis 95 Vol.-% umfasst. Die Erfinder haben festgestellt, dass sich Ausgangsmaterialien auch mit nur relativ geringen Konzentrationen an Alkoholen gut umsetzen lassen, z.B. im Bereich von 10 bis 500 g/l, besonders bevorzugt im Bereich von 15 bis 300 g/l).
Der Wassergehalt der Lösung ist nicht kritisch und kann in weiten Bereichen schwanken. Es ist aber festzuhalten, dass er ggf. relativ gering sein kann, weil katalytische Mengen bereits ausreichend sind. In der Regel sollte er jedoch mindestens 1 oder besser 2 Gew.-% der Lösung ausmachen. Die wässrige Alkohol-Lösung kann weitere flüssige (gelöste) kohlenstoffhaltige Bestandteile wie organische Säuren enthalten, bei denen es sich z.B. um Nebenprodukte einer fermentativen oder petrochemischen Vorreaktion handeln kann. Dabei sind die Art dieser Bestandteile sowie der Gesamt-Kohlenstoffgehalt in der Lösung nicht beschränkt.
Die Kontaktzeit des Alkohols mit dem Katalysator und damit die Verweilzeit unter den angegebenen Bedingungen beträgt zwischen 5 und 300 s. Geringere Konzentrationen verkürzen tendenziell die Verweildauer noch weiter, so dass sich das Verfahren mit Kontakt-/Verweilzeiten von in der Regel nicht mehr als 100 s durchführen lässt, wobei gleichzeitig überragende Ergebnisse sowohl hinsichtlich des Gesamtumsatzes als auch der Spezifität zu beobachten sind. Besonders günstig sind Verweilzeiten von 10 bis 80 s, darunter nochmals besonders bevorzugt von 20 bis 35 s, und dies insbesondere in Kombination mit den vorgenannten Drücken und/oder Temperaturen. Diese geringe Verweildauer ist es insbesondere, die es ermöglicht, dass das Verfahren als kontinuierliches Verfahren mit einem hohen Durchsatz ausgestaltet werden kann.
Die Trennung von Gas- und Flüssigphase wird vorzugsweise noch unter Druck durchgeführt. Damit steht das Olefin nach der Reaktion noch unter Druck und kann so weiter umgesetzt oder abgefüllt werden.
Sofern restlicher Alkohol sowie ggf. gebildeter Dialkylether in der Flüssigphase verblieben sind, kann der Produktstrom zur Erreichung eines nochmals verbesserten Umsatzes nochmals in den Reaktor zurückgeführt werden, beispielsweise in Form einer Beimischung zu frischem Ausgangsmaterial.
Das erfindungsgemäße Verfahren eignet sich insbesondere zur Verwertung von fermentativ behandelten Biomaterialien oder alkoholhaltigen Lösungen, die in der chemischen Industrie (z.B. in der Petrochemie) anfallen. Gegebenenfalls vorhandene Feststoffe wie cellulosische Materialien sollten zuvor abgetrennt worden sein, um eine unnötige Verkokung und damit Inaktivierung des Katalysators zu vermeiden. Das Mittel der Wahl hierfür ist häufig eine einstufige Verdampfung (Flashdestillation). Gelöste Salze stören in der Regel nicht, solange sich unter den Reaktionsbedingungen ein saurer pH-Wert einstellt, gehen aber bereits bei einer einstufigen Verdampfung im Wesentlichen nicht mit über. In dieser Hinsicht hat das erfindungsgemäße Verfahren einen deutlichen Vorteil gegenüber der früher üblichen Gasphasendehydratisierung, weil für diese die Aufreinigung des Ausgangsgemischs durch eine aufwändige Rektifikation erfolgen muss.

Gegenüber der klassischen Dehydratisierung in der Gasphase ergeben sich damit die folgenden Vorteile der Erfindung:
- Einsparung der mehrstufigen Rektifikation zur Aufkonzentrierung fermentativ hergestellter Alkohole
- Verbesserung der Raum-Zeit-Ausbeute aufgrund der Reaktion in komprimierter Phase
- Geringerer Prozessenergiebedarf, da kein Verdampfen notwendig, nur Erhitzen
- Das Olefin steht nach der Reaktion unter hohem Druck zur Verfügung - Einsparung einer Gaskompression
- Wegfall der Transport- und Lagerkosten für z.B. Ethylen, da direkt beim Verbraucher (z.B. in räumlicher Nähe zu Fabrikationsanlagen, die keinem Pipelineverbund angehören und damit keinen direkten Zugang zu z.B. Ethylen oder Propylen haben) produziert werden kann.
- Der entwickelte Prozess kann in kleinen Anlagen realisiert werden, so dass kein Pipeline-Anschluss erforderlich ist.

Eigene Versuche der Erfinder der vorliegenden Anmeldung mit flüssigen Säuren führten zu Ergebnissen, die teilweise in diametralem Gegensatz zu den Ergebnissen der Literatur stehen, zumindest jedoch nicht zu erwarten waren, insbesondere in Hinblick auf das Verhältnis von Ausbeute und Spezifität (siehe z.B. das (nicht die Erfindung darstellende) Beispiel 2, demgemäß Schwefelsäure als Katalysator eine wesentlich geringere Spezifität für die Konversion von Ethanol besitzt als für die Konversion von Propanol publiziert). Hier könnte möglicherweise das ebenfalls im Stand der Technik angesprochene Problem der Aggressivität von Schwefelsäure und deren Auswirkung auf die Behälterwände eine große Rolle spielen. Da Watanabe et al. die Propanol-Dehydratisierung sowohl mit flüssiger Säure als auch mit festen Oxiden untersuchten und die Ergebnisse bei Einsatz fester Katalysatoren geringfügig schlechter als die mit Säuren erzielten waren, konnte man auch nicht erwarten, dass die Dehydratisierung der "harten Nuss" Ethanol an sauren Festkörper-Katalysatoren erfolgreich ablaufen würde. Die Erfinder der vorliegenden Anmeldung haben jedoch festgestellt, dass der Umsatz von Ethanol bei Verwendung von Schwefelsäure und von festen Katalysatoren in etwa gleich und in jedem Fall sehr hoch ist und die Spezifität - und damit die selektive Ausbeute an Ethylen - bei Verwendung von Aluminiumoxid jedoch um mehr als das 10-fache steigt. Außerdem konnten sie feststellen, dass Reaktionsbedingungen mit Drücken/Temperaturen deutlich im überkritischen Bereich, z.B. Temperaturen von ≥ 380°C und/oder Drücken von ≥ 240 bar den Umsatz deutlich steigern, während die Selektivität etwas sinkt. Eine weitere Steigerung des Umsatzes gelingt, wenn anstelle von Aluminiumoxid Zirkoniumdioxid oder Titandioxid verwendet werden. Der Einsatz säurenmodifizierter Katalysatoren bewirkt eine hohe Ethylen-Selektivität.

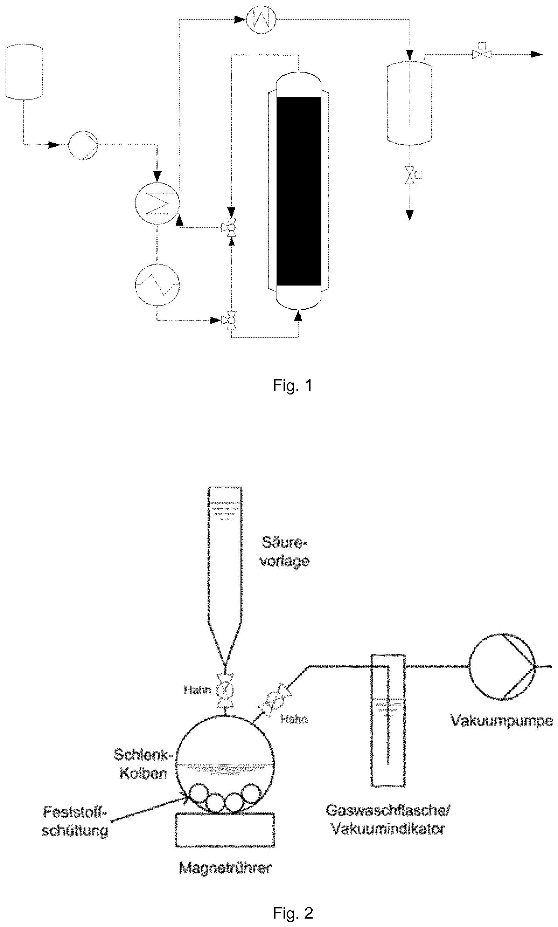

Diese Ergebnisse waren völlig überraschend. Erst diese Erkenntnis macht jedoch eine erfolgreiche Dehydratisierung in Form eines kontinuierlichen Verfahrens an einem Festbettkatalysator möglich. In bevorzugter Weise verläuft die Umsetzung wie folgt, wobei ein hierfür geeignetes Anlagenschema in **Figur 1** gezeigt ist: Eine wässrige alkoholische Lösung, die z.B. durch fermentative oder petrochemische Prozesse gewonnen wurde, wird zunächst aus einer Vorlage wie dem Vorlagebehälter 1 mittels einer Pumpe (hier einer Hochdruckpumpe 2) auf mindestens 220 bar oder einen der oben genannten bevorzugten Druckbereiche verdichtet. Die Lösung wird auf Temperaturen zwischen vorzugsweise 350 bis 600°C gebracht, was mit Hilfe eines Gegenstromwärmetauschers 3 und/oder eines Überhitzers 4 bewirkt werden kann. Zum Anfahren der Anlage kann z.B. der Bypass 5 genutzt werden. Die überkritische Reaktionslösung wird in einem Reaktor 6, der adiabat oder isotherm betrieben werden kann, an einem Katalysatorfestbett, z.B. einer Schüttung, umgesetzt. Natürlich wäre auch die Verwendung eines monolithischen Katalysatormaterials möglich. Das Katalysatorfestbett, in der Regel wie technisch üblich geschüttet, füllt vorzugsweise das gesamte Reaktorvolumen. Dies hat mehrere Vorteile. Zum einen erzielt man auf diese Weise ein gleichmäßiges Durchströmen des Reaktors. Zum anderen ist die Reaktionslösung während der gesamten Verweildauer im Reaktor dem Kontakt mit dem Katalysator unter den dort herrschenden Druck- und Temperaturbedingungen ausgesetzt.

Alternativ kann z.B. auch eine Reaktorkaskade zur Selektivitätssteigerung verwendet werden. Die Verweilzeit im Reaktor beträgt hier 5 bis 300 s. Als Katalysator können die vorgenannten Katalysatoren eingesetzt werden, ggf. auch als Mischungen oder säuremodizifierte Trägermaterialien. Nach Verlassen des Reaktorteils gelangt der Produktstrom vorzugsweise in einen Kühler 7, in günstiger Weise über den genannten Gegenstromwärmetauscher. In dem Kühler erfolgt eine Abkühlung, z.B. auf unter 50°C, wobei der Druck jedoch aufrechterhalten wird. Vom Kühler wird das zweiphasige Gemisch in einen Abscheider 8 geführt, in dem die Trennung des Gemischs in eine Roh-Olefin-Gasphase 9 und eine wässrige Flüssigphase 10 erfolgt. Die Gasphase kann im Anschluss einer weiteren Aufreinigung unterzogen werden, wie aus dem Stand der Technik bekannt.
Die vorgenannte Umsetzung wurde unter anderem an den folgenden Katalysatoren durchgeführt, die durch ein Tränkverfahren hergestellt wurden:
Kat 01 Aktivkohle + H₂SO₄ (unter Stickstoffatmosphäre kalziniert)
Kat 02 Aktivkohle + H₃PO₄ (unter Stickstoffatmosphäre kalziniert)
Kat 03 Al₂O₃ + H₂SO₄
Kat 04 Al₂O₃ + H₃PO₄
Kat 05 TiO₂ + H₂SO₄
Kat 06 TiO₂ + H₃PO₄
Kat 07 ZrO₂ + HₛSO₄
Kat 08 ZrO₂ + H₃PO₄

Diese Katalysatoren können beispielsweise nach dem nachstehend beschriebenen Verfahren und/oder mit Hilfe der in **Figur 2** dargestellten Vorrichtung hergestellt werden. Dabei wird ein Kolben - hier ein Schlenkkolben - mit einer Vakuumpumpe evakuiert, um sicherzustellen, dass vor dem Tränken des Feststoffs mit Säuren sämtlicher kapillarer Sauerstoff aus dem Katalysatormaterial entfernt wurde. Als Vakuumindikator dient eine mit Wasser befüllte Gaswaschflasche. Erlahmt die Gasblasenbildung am Tauchrohr der Gaswaschflasche, kann sichergestellt werden, dass das maximal mögliche Vakuum erreicht ist. Weiter unterbindet das Wasserbad ein Austreten feinster Partikel. Nach Erreichen des Vakuums kann die Säurevorlage in das Reaktionsgefäß eingelassen werden; das Vakuum sollte bestehen bleiben. Durch Dispergieren des Katalysator-Feststoffs in Schwefelsäure (z.B. 1N) oder Phosphorsäure (z.B. 1N) werden aktive schwefel- oder phosphorsaure Zentren auf den Partikeloberflächen erzeugt. Nach einer Einwirkdauer, die z.B. zwischen 0,5 und 2 Stunden liegen kann und vorzugsweise bei ca. 1 Stunde liegt, kann das Vakuum aufgehoben und der Kolbeninhalt über einen Filter (z.B. einen Blaubandfilter) entleert werden. Der Filterrückstand wird getrocknet, z.B. für 24 Stunden bei ca. 105°C im Trockenschrank, und anschließend kalziniert, beispielsweise für 24 Stunden bei 500°C.

Zur Herstellung von Metall- und Halbmetallphosphaten werden die entsprechenden Metall- oder Halbmetallverbindungen, z.B. Oxide, Säuren, Chloride oder Oxichloride mit Phosphorsäure umgesetzt. Beispiele für geeignete Kationen sind Titan, Zirkonium und Aluminium. Für Reaktionen im Labormaßstab können hierfür in günstiger Weise ca. 5 bis 50 g der jeweiligen Metall- oder Halbmetallverbindung eingewogen und in ein Reaktionsgefäß überführt werden. Dazu wird eine überstöchiometrische Masse an 85%iger Orthophosphorsäure (oder einer anderen Phosphorsäure) zugegeben, und das Reaktionsgemisch wird für eine bessere Homogenität und Durchmischbarkeit zusätzlich mit deionisiertem Wasser (vorzugsweise in einer Menge von maximal 50% der Phosphorsäure) versehen. Das Reaktionsgefäß sowie auch die Reaktionsführung unterscheiden sich für exotherme und endotherme Reaktionen. Endotherme Reaktionen werden in einem beheizten Rundkolben im Siedezustand mit Rückflusskühlung, exotherme Reaktionen in einem gekühlten Becherglas durchgeführt. Die Siedetemperatur liegt bei zwischen etwa 90°C und 110°C. Die Reaktionsdauer für die chemische Umsetzung zu den Phosphaten beträgt hierbei in günstiger Weise jeweils etwa 4 Stunden im gewählten Temperaturbereich.

Nach Ablauf der Reaktionsdauer werden die Lösungen auf Raumtemperatur temperiert. Je nach Art des Anions des Edukts kann eine zweite Reaktion zur Neutralisation entstehender anorganischer Säure und gleichzeitiger Fällung des Phosphats nötig sein. Beispielsweise wird bei der Verwendung eines chloridhaltigen Ausgangsmaterials entstehende Salzsäure mit NaOH neutralisiert. Anschließend wird das Produkt z.B. über einen Filter für feine Niederschläge vakuumfiltriert. Vorzugsweise wird hier ein Blaubandfilter verwendet.

Der so erhaltene Filterrückstand wird von Oberflächenwasser befreit, z.B. für 24 Stunden bei 105°C im Trockenschrank. Vorzugsweise wird das Phosphat anschließend kalziniert, z.B. bei erhöhter Temperatur in einem Muffelofen. Dabei wird Kapillar- und Kristallwasser entfernt, wodurch der Katalysator seine Aktivität erhält. Anhand von Messungen der spezifischen Oberfläche und deren Korrelation mit der katalytischen Aktivität hat sich hierfür ein Temperaturbereich von zwischen etwa 300°C und 1100°C als besonders günstig erwiesen. Die Qualität der Katalysatorpräparation kann z.B. gravimetrisch bestimmt werden. Auch der Prozentsatz der Belegung der Oberfläche mit sauren Gruppen, relativ zu der maximal möglichen Belegung, kann ggf. bestimmt werden. Das häufig beim Kalzinieren stückig gewordene Material wird anschließend in einem Mörser zerkleinert.

Die Erfindung soll nachstehend anhand von Beispielen näher erläutert werden.

### Beispiel 1 (Vergleichsbeispiel)

In einer Anlage wie in Fig. 1 dargestellt wurde der Reaktor vollständig mit 150 mL SiC als Trägermaterial befüllt, um ein gleichbleibendes Strömungsprofil zu erzielen. Als Ausgangsmaterial wurde eine 25%ige ethanolische Lösung in Wasser bei einer Reaktionstemperatur von 400°C und einem Druck von 250 bar umgesetzt. Die Verweilzeit im Reaktor betrug 20s. Dabei wurde ein Umsatz von 21,8% bei einer Ethylen-Selektivität von 29,7% erreicht.

### Beispiel 2 (Vergleichsbeispiel)

Beispiel 1 wurde mit der Änderung wiederholt, dass der ethanolischen Lösung vor Eintritt in den Reaktor einige Tropfen 98 gew.-%ige Schwefelsäure zugesetzt Dabei wurde ein Umsatz von 89,7% bei einer Ethylen-Selektivität von 6,8% erreicht.

### Beispiel 3

Beispiel 1 wurde mit der Änderung wiederholt, dass der Reaktor anstelle von SiC als Trägermaterial γ-Al₂O₃-Pellets enthielt. Dabei wurde ein Umsatz von 23,3% bei einer Ethylen-Selektivität von 91,5% erreicht.

### Beispiel 4

Beispiel 3 wurde mit der Änderung wiederholt, dass die Verweildauer 30 s betrug. Dabei wurde ein Umsatz von 92,5% bei einer Ethylen-Selektivität von 97,6% erreicht.

### Beispiel 5:

In einer Anlage wie in Fig. 1 dargestellt wurde der Reaktor mit 44 mL ZrO₂ (tetragonal) als Katalysatormaterial befüllt. Als Ausgangsmaterial wurde eine 39%ige ethanolische Lösung in Wasser bei einer Reaktionstemperatur von 357°C und einem Druck von 230 bar umgesetzt. Die Verweilzeit im Reaktor betrug 64s. Dabei wurde ein Umsatz von 56,7% bei einer Ethylen-Selektivität von 73,8% erreicht.

### Beispiel 6:

In einer Anlage wie in Fig. 1 dargestellt wurde der Reaktor mit 28 mL γ-Al₂O₃ als Katalysatormaterial befüllt. Als Ausgangsmaterial wurde eine 39%ige ethanolische Lösung in Wasser bei einer Reaktionstemperatur von 357°C und einem Druck von 230 bar umgesetzt. Die Verweilzeit im Reaktor betrug 75s. Dabei wurde ein Umsatz von 47,9 % bei einer Ethylen-Selektivität von 70,4% erreicht.

### Beispiel 7:

In einer Anlage wie in Fig. 1 dargestellt wurde der Reaktor mit 33 mL TiO₂ (Anatas) als Katalysatormaterial befüllt. Als Ausgangsmaterial wurde eine 39%ige ethanolische Lösung in Wasser bei einer Reaktionstemperatur von 357°C und einem Druck von 230 bar umgesetzt. Die Verweilzeit im Reaktor betrug 72s. Dabei wurde ein Umsatz von 87,9 % bei einer Ethylen-Selektivität von 11,5% erreicht.

## Patentansprüche

1. Verfahren zur kontinuierlichen Herstellung eines oder mehrerer Olefine aus einer wässrigen Lösung des oder der entsprechenden Alkohole, wobei die Alkohole ausgewählt sind unter Ethanol, Propanol und Butanol, umfassend die Schritte:
Bereitstellen einer feststofffreien wässrigen Lösung des oder der Alkohole,
Führen dieser Lösung durch einen Reaktor, der mit einem Festbettkatalysator derart befüllt ist, dass die Lösung beim Durchströmen des Reaktors mit dem Katalysator in Kontakt kommt, bei einer Temperatur von mindestens 300°C und einem Druck von mindestens 220 bar in einer solchen Temperatur-Druck-Kombination, dass der oder die Alkohole unter überkritischen Bedingungen umgesetzt werden, wobei die Verweildauer der Lösung im Reaktor zwischen 5 und 300 Sekunden beträgt, und
Überführen des entstandenen zweiphasigen Gemischs in einen Abscheider, in dem die Trennung des Gemischs in eine Roh-Olefin-Gasphase und eine wässrige Flüssigphase erfolgt,
wobei der Katalysator ausgewählt ist unter Metalloxiden, die die Eigenschaften einer Brønsted-Säure haben, unlöslichen Metall- oder Halbmetallphosphaten sowie porösen Materialien, ausgewählt unter Bimsstein und Kohlen, deren Oberfläche mit anorganischen Säuregruppen belegt ist, wobei die Metalloxide Oxide von Kationen der 3. Hauptgruppe sowie der 4. bis 6. Nebengruppe der Periodensystems sind und wobei der Ausdruck "Metalloxide" unmodifizierte und mit Säuregruppen modifizierte Materialien bezeichnet.

2. Verfahren nach Anspruch 1, **dadurch gekennzeichnet, dass** die Verweildauer der Lösung im Reaktor zwischen 5 und 100 Sekunden und bevorzugt zwischen 10 und 80 Sekunden beträgt.

3. Verfahren nach einem der Ansprüche 1 oder 2, **dadurch gekennzeichnet, dass** der Reaktor vollständig mit einer Katalysatorschüttung befüllt ist.

4. Verfahren nach einem der Ansprüche 1 bis 3, **dadurch gekennzeichnet, dass** die Oberfläche des Katalysators anorganische Säuregruppen aufweist.

5. Verfahren nach einem der voranstehenden Ansprüche, **dadurch gekennzeichnet, dass** der Festbettkatalysator ausgewählt ist unter Oxiden von Kationen der 3. Hauptgruppe, sowie der 4. bis 6. Nebengruppe des Periodensystems, deren Oberfläche mit anorganischen Säuregruppen belegt ist.

6. Verfahren nach einem der voranstehenden Ansprüche, **dadurch gekennzeichnet, dass** der Festbettkatalysator ausgewählt ist unter Oxiden des Aluminiums, des Zirkoniums und des Titans, deren Oberfläche bevorzugt mit anorganischen Säuregruppen belegt ist, porösen Materialien, ausgewählt unter Bimsstein oder Kohlen, deren Oberfläche mit Sulfat- oder Phosphatgruppen belegt ist, und Metallphosphaten.

7. Verfahren nach einem der voranstehenden Ansprüche, **dadurch gekennzeichnet, dass** der Festbettkatalysator ausgewählt ist unter Aluminiumphosphat, Zirkoniumphosphat, phosphatiertem Aluminiumoxid, phosphatiertem Titanoxid, phosphatiertem Zirkoniumoxid, phosphatierter Kohle, phosphatiertem Bims, sulfatiertem Zirkoniumoxid, sulfatiertem Titanoxid, sulfatierter Kohle, sulfatiertem Bims und sulfatiertem Aluminiumoxid.

8. Verfahren nach einem der voranstehenden Ansprüche, **dadurch gekennzeichnet, dass** die wässrige Lösung des oder der Alkohole Ethanol, Propanol oder Butanol als einzigen Alkohol oder als einen der Alkohole in einer Menge von mindestens 10 Gew.-%, bevorzugt mindestens 20 Gew.-%, stärker bevorzugt mindestens 50 Gew.-%, bezogen auf das Gesamtgewicht der vorhandenen Alkohole, aufweist.

9. Verfahren nach einem der voranstehenden Ansprüche, **dadurch gekennzeichnet, dass** die wässrige Lösung des oder der Alkohole Ethanol als einzigen Alkohol oder als einen der Alkohole in einer Menge von mindestens 10 Gew.-%, bevorzugt mindestens 20 Gew.-%, stärker bevorzugt mindestens 50 Gew.-%, bezogen auf das Gesamtgewicht der vorhandenen Alkohole, aufweist.

10. Verfahren nach einem der voranstehenden Ansprüche, **dadurch gekennzeichnet, dass** die Temperatur mindestens 350°C und vorzugsweise 400°C oder mehr und/oder der Druck vorzugsweise 250 bar oder mehr beträgt.

11. Verfahren nach einem der voranstehenden Ansprüche, **dadurch gekennzeichnet, dass** die Ausgangskonzentration des oder der Alkohole in der wässrigen Lösung 5 bis 95% (Gew./Vol.), vorzugsweise 15 bis 30% (Gew./Vol.) beträgt.

12. Verfahren nach einem der voranstehenden Ansprüche, **dadurch gekennzeichnet, dass** die wässrige Lösung nach Verlassen des den Festbettkatalysator enthaltenden Reaktors noch unter Druck stehend abgekühlt wird, worauf eine Trennung von Gas- und Flüssigphase erfolgt derart, dass das entstandene Olefin weiterhin unter Druck gehalten wird.

13. Verfahren nach einem der voranstehenden Ansprüche, **dadurch gekennzeichnet, dass** die entstandene wässrige Flüssigphase nach Abtrennung von der Roh-Olefin-Gasphase mit einer frischen Lösung des oder der Alkohole gemischt und zusammen mit dieser wiederum in den Reaktor geführt wird, wobei die Verweildauer der Lösung im Reaktor zwischen 5 und 100 Sekunden und vorzugsweise zwischen 10 und 80 Sekunden beträgt.

14. Verfahren nach einem der voranstehenden Ansprüche, **dadurch gekennzeichnet, dass** als alkoholische wässrige Lösung eine Lösung eingesetzt wird, die aus fermentativ behandelten Biomaterialien oder alkoholhaltigen Materialien, die in der chemischen Industrie, vorzugsweise in der Petrochemie anfallen, gewonnen wurde.

15. Verfahren nach einem der voranstehenden Ansprüche, **dadurch gekennzeichnet, dass** die Erwärmung der wässrigen alkoholischen Lösung mit Hilfe eines Gegenstromwärmetauschers erfolgt, in dem die alkoholische Lösung im Gegenstrom zu der den Reaktor verlassenden Lösung geführt wird.

## Claims

1. A process for the continuous production of one or more olefins from an aqueous solution of corresponding alcohol(s), wherein said alcohols are selected from among ethanol, propanol, and butanol, comprising the following steps:
providing a solid-free aqueous solution of said alcohol(s),
putting said solution through a reactor, which is filled with a fixed-bed catalyst such that said solution comes into contact with the catalyst when flowing through said reactor, at a temperature of at least 300°C and a pressure of at least 220 bar in such a temperature/pressure combination that said alcohol(s) are reacted under supercritical conditions, wherein the retention time of the solution in the reactor is between 5 and 300 seconds, and
transferring a resulting two-phase mixture to a separator, in which said mixture is separated in a raw olefin gas phase and an aqueous liquid phase,
wherein said catalyst is selected from among oxides of metals having the properties of a Bronsted acid, insoluble metallic or semi-metallic phosphates as well as porous materials selected from among pumice and carbon, the surface area of which is coated with inorganic acid groups, wherein the metal oxides are oxides of cations of the third main group as well as the fourth and sixth secondary group of the periodic table, and wherein the term "metal oxide" denotes unmodified materials and materials modified with acid groups.

2. The process according to claim 1, wherein the retention time of said solution in said reactor is between 5 and 100 seconds, and preferably between 10 and 80 seconds.

3. The process according to claim 1 or 2, wherein said reactor is completely filled with a catalyst fill.

4. The process according to any one of claims 1 to 3, wherein the surface area of said catalyst has inorganic acid groups.

5. The process according to any one of the preceding claims, wherein said fixed-bed catalyst is selected from among oxides of cations of the third main group as well as fourth to sixth secondary group of the periodic table, the surface area of which is coated with inorganic acid groups.

6. The process according to any one of the preceding claims, wherein said fixed-bed catalyst is selected from among oxides of aluminum, zirconium, and titanium, the surface area of which is preferably coated with inorganic acid groups, porous materials selected from among pumice and carbon, the surface area of which is coated with sulfate or phosphate groups and metal phosphates.

7. The process according to any one of the preceding claims, wherein said fixed-bed catalyst is selected from among aluminum phosphate, zirconium phosphate, phosphated aluminum oxide, phosphated titanium oxide, phosphated zirconium oxide, phosphated carbon, phosphated pumice, sulfated zirconium oxide, sulfated titanium oxide, sulfated carbon, sulfated pumice, and sulfated aluminum oxide.

8. The process according to any one of the preceding claims, wherein said aqueous solution of the alcohol(s) has ethanol, propanol or butanol as a single alcohol or as one of the alcohols in a quantity of at least 10% by weight, preferably at least 20% by weight, more preferably at least 50% by weight, in relation to the overall weight of available alcohols.

9. The process according to any one of the preceding claims, wherein said aqueous solution of the alcohol(s) has ethanol as a single alcohol or as one of the alcohols in a quantity of at least 10% by weight, preferably at least 20% by weight, more preferably at least 50% by weight, in relation to the overall weight of available alcohols.

10. The process according to any one of the preceding claims, wherein said temperature is at least 350°C and preferably 400°C or more and/or said pressure is preferably 250 bar or more.

11. The process according to any one of the preceding claims, wherein an initial concentration of the alcohol(s) in said aqueous solution is 5 to 95% (wt./vol.), preferably 15 to 30% (wt./vol.).

12. The process according to any one of the preceding claims, wherein said aqueous solution is cooled after leaving the reactor containing said fixed-bed catalyst while still under pressure, whereupon a gas and liquid phase is separated such that the resulting olefin continues to remain under pressure.

13. The process according to any one of the preceding claims, wherein said resulting aqueous liquid phase is mixed with a new solution of said alcohol(s) after being separated from the raw olefin gas phase and is in turn led to the reactor together with it, wherein the retention time of said solution in the reactor is between 5 and 100 seconds and preferably between 10 and 80 seconds.

14. The process according to any one of the preceding claims, wherein a solution is used as an alcoholic aqueous solution, which was obtained from biomaterials or materials containing alcohol treated through fermentation that arise in the chemical industry, preferably in the petrochemical industry.

15. The process according to any one of the preceding claims, wherein said aqueous alcoholic solution is heated with the help of a counter-current heat exchanger, in which the alcoholic solution is fed to the solution leaving the reactor in a counter-flow.

## Revendications

1. Procédé de production en continu d'une ou de plusieurs oléfines à partir d'une solution aqueuse du ou des alcools correspondants, dans lequel les alcools sont sélectionnés parmi l'éthanol, le propanol et le butanol, comprenant les étapes suivantes:
préparer une solution aqueuse dépourvue de substances solides du ou des alcools,
conduire cette solution à travers un réacteur, qui est rempli avec un catalyseur à lit fixe, de telle manière que la solution vienne en contact avec le catalyseur lorsqu'elle traverse le réacteur, à une température d'au moins 300°C et sous une pression d'au moins 220 bar, avec une combinaison température-pression telle que le ou les alcools réagissent en conditions supercritiques, dans lequel la durée de séjour de la solution dans le réacteur est comprise entre 5 et 300 secondes, et
transférer le mélange diphasique obtenu dans un séparateur, dans lequel le mélange est séparé en une phase gazeuse oléfinique brute et une phase liquide aqueuse,
dans lequel le catalyseur est choisi parmi des oxydes métalliques qui présentent les propriétés d'un acide de Brønsted, des phosphates métalliques ou hémimétalliques insolubles ainsi que des matériaux poreux, choisis parmi la pierre ponce et des charbons, dont la surface porte des groupes acides inorganiques, dans lequel les oxydes métalliques sont des oxydes de cations du 3^{ème} groupe principal ainsi que du 4^{ème} au 6^{ème} groupe secondaire du système périodique et dans lequel l'expression "oxydes métalliques" désigne des matériaux non modifiés et des matériaux modifiés avec des groupes acides.

2. Procédé selon la revendication 1, **caractérisé en ce que** la durée de séjour de la solution dans le réacteur est comprise entre 5 et 100 secondes et de préférence entre 10 et 80 secondes.

3. Procédé selon une des revendications 1 ou 2, **caractérisé en ce que** le réacteur est entièrement rempli avec une charge de catalyseur en vrac.

4. Procédé selon l'une quelconque des revendications 1 à 3, **caractérisé en ce que** la surface du catalyseur présente des groupes acides inorganiques.

5. Procédé selon l'une quelconque des revendications précédentes, **caractérisé en ce que** le catalyseur à lit fixe est choisi parmi des oxydes de cations du 3^{ème} groupe principal, ainsi que du 4^{ème} au 6^{ème} groupe secondaire du système périodique, dont la surface porte des groupes acides inorganiques.

6. Procédé selon l'une quelconque des revendications précédentes, **caractérisé en ce que** le catalyseur à lit fixe est choisi parmi des oxydes de l'aluminium, du zirconium et du titane, dont la surface porte de préférence des groupes acides inorganiques, des matériaux poreux, choisis parmi la pierre ponce ou des charbons, dont la surface porte des groupes sulfate ou phosphate, et des phosphates métalliques.

7. Procédé selon l'une quelconque des revendications précédentes, **caractérisé en ce que** le catalyseur à lit fixe est choisi parmi le phosphate d'aluminium, le phosphate de zirconium, l'oxyde d'aluminium phosphaté, l'oxyde de titane phosphaté, l'oxyde de zirconium phosphaté, le charbon phosphaté, la pierre ponce phosphatée, l'oxyde de zirconium sulfaté, l'oxyde de titane sulfaté, le charbon sulfaté, la pierre ponce sulfatée et l'oxyde d'aluminium sulfaté.

8. Procédé selon l'une quelconque des revendications précédentes, **caractérisé en ce que** la solution aqueuse du ou des alcools présente l'éthanol, le propanol ou le butanol comme alcool unique ou comme un des alcools en une quantité d'au moins 10 % en poids, de préférence d'au moins 20 % en poids et de préférence encore d'au moins 50 % en poids, rapporté au poids total des alcools présents.

9. Procédé selon l'une quelconque des revendications précédentes, **caractérisé en ce que** la solution aqueuse du ou des alcools présente l'éthanol comme alcool unique ou comme un des alcools en une quantité d'au moins 10 % en poids, de préférence d'au moins 20 % en poids et de préférence encore d'au moins 50 % en poids, rapporté au poids total des alcools présents.

10. Procédé selon l'une quelconque des revendications précédentes, **caractérisé en ce que** la température vaut au moins 350°C et de préférence 400°C ou plus et/ou la pression vaut de préférence 250 bar ou plus.

11. Procédé selon l'une quelconque des revendications précédentes, **caractérisé en ce que** la concentration initiale du ou des alcools dans la solution aqueuse vaut 5 à 95 % (poids/volume), de préférence 15 à 30 % (poids/volume).

12. Procédé selon l'une quelconque des revendications précédentes, **caractérisé en ce que** l'on refroidit la solution aqueuse après qu'elle ait quitté le réacteur contenant le catalyseur à lit fixe alors qu'elle se trouve encore sous pression, puis on opère une séparation de phase gazeuse et phase liquide, de telle manière que l'oléfine obtenue soit encore maintenue sous pression.

13. Procédé selon l'une quelconque des revendications précédentes, **caractérisé en ce que** l'on mélange la phase liquide aqueuse obtenue après la séparation de la phase gazeuse oléfinique brute avec une solution fraîche du ou des alcools et on la renvoie avec celle-ci dans le réacteur, dans lequel la durée de séjour de la solution dans le réacteur est comprise entre 5 et 100 secondes et de préférence entre 10 et 80 secondes.

14. Procédé selon l'une quelconque des revendications précédentes, **caractérisé en ce que** l'on utilise comme solution aqueuse alcoolique une solution, qui a été obtenue à partir de biomatériaux traités par fermentation ou de matériaux contenant de l'alcool, qui se forment dans l'industrie chimique, de préférence dans la pétrochimie.

15. Procédé selon l'une quelconque des revendications précédentes, **caractérisé en ce que** l'on effectue le chauffage de la solution alcoolique aqueuse à l'aide d'un échangeur de chaleur à contre-courant, dans lequel la solution alcoolique est conduite à contre-courant de la solution quittant le réacteur.
